# EUROPEAN PATENT APPLICATION

(11) **EP 4 043 550 A2**
(43) Date of publication of application: **17.08.2022**
(21) Application number: 20874292.4
(22) Date of filing: 08.10.2020
(51) Int. Cl.: C12N 5/00, C12N 5/0775, C12N 5/071, A61K 35/28, A61K 48/00

(54) **COMPOSITION FOR AUGMENTING STEMNESS AND USE THEREOF**

(30) Priority: 08.10.2019 KR 20190124769
(71) Applicant: Genechem Inc., Daejeon 34025 (KR); University Industry Foundation, Yonsei University Wonju Campus, Gangwon-do 26493 (KR); Industry-Academic Cooperation Foundation, Yonsei University, Seoul 03722 (KR)
(72) Inventor: KIM, Sung Hoon, Wonju-si, Gangwon-do 26399 (KR); BAEK, Ah Reum, Seoul 03776 (KR); CHO, Sung Rae, Seoul 04428 (KR); WOO, Jin Suk, Daejeon 34049 (KR); KIM, Li La, Yeongdong-gun, Chungcheongbuk-do 29121 (KR); KIM, Dae Hee, Daejeon 35265 (KR)
(74) Representative: J A Kemp LLP
(86) International application number: PCT/KR2020/013745
(87) International publication number: WO 2021/071289

(57) **Abstract**

The present application pertains to a sialyloligosaccharide for augmenting the stemness of stem cells and a use thereof and provides a composition for augmenting stemness of stem cells, a method of culturing stem cells in a medium containing a sialyloligosaccharide, a method of augmenting stemness of stem cells, a stem cell with augmented stemness, obtained by the method, and a cell therapy composition including the stem cells as an active ingredient. The composition or the method according to one or more embodiments may suppress the aging of stem cells and may maintain and augment stemness.

<110>     GENECHEM INC.
          UNIVERSITY INDUSTRY FOUNDATION, YONSEI UNIVERSITY WONJU CAMPUS

<120>     Compositions for enhancing stemness and uses thereof

<130>     PX063067PCT

<150>     KR 10-2019-0124769
<151>     2019-10-08

<160>     4

<170>     KoPatentIn 3.0

<210>     1
<211>     19
<212>     DNA
<213>     Artificial Sequence

<220>
<223>     OCT4 forward


<400>     1
gcaagccctc atttcacca                                                    19


<210>     2
<211>     18
<212>     DNA
<213>     Artificial Sequence

<220>
<223>     OCT4 reverse


<400>     2
gcccatcacc tccaccac                                                     18


<210>     3
<211>     21
<212>     DNA
<213>     Artificial Sequence

<220>
<223>     NANOG forward


<400>     3
tttgtgggcc tgaagaaaac t                                                 21


<210>     4
<211>     21
<212>     DNA
<213>     Artificial Sequence

<220>
<223>     NANOG reverse


<400>     4

agggctgtcc tgaataagca g 21

## Description

### TECHNICAL FIELD

The inventive concept relates to a composition for augmenting stemness and a use thereof.

### BACKGROUND ART

Various types of cells are being researched and developed to use them as cellular therapeutic agents, and pluripotent stem cells (embryonic stem cells, induced pluripotent stem cells, etc.), multipotent stem cells, or adult stem cells (bone marrow/fat-derived stem cells, umbilical cord blood/umbilical cord stem cells, fetal stem cells, etc.), and somatic cells are being used as the cells according to the differentiation sites. Among these, human embryonic stem cells have many ethical problems because they are made from embryos that can develop into human organisms, and the differentiation control technology of pluripotent stem cells have not yet been completed, so it is known to take a long time to be developed as a cellular therapeutic agents. Also, although the research and development of cellular therapeutic agents using somatic cells has shown many results, it is difficult to put them into practical use due to the low proliferation inherent in somatic cells and the difficulty in securing raw material tissues. Therefore, much attention is focused on the study of adult stem cells as an alternative to overcome these problems.

Among adult stem cells, mesenchymal stem cells have superior proliferative capacity compared to somatic cells and are multipotent stem cells capable of differentiating into bone, cartilage, and fat. Mesenchymal stem cells are genetically stabilized more than pluripotent stem cells such as embryonic stem cells and thus have been developed as cellular therapeutic agents for cartilage regeneration, myocardial infarction treatment, and graft-versus-host disease. Although it is true that mesenchymal stem cells show higher self-reproduction capacity than somatic cells, unlike when in the body, the best conditions for maintaining the proliferation of cells must be provided in vitro culture conditions, and it is difficult to meet environmental conditions such as nutrients, pH, temperature, and osmotic pressure that are close to the living conditions. In particular, there is a problem in that aging is caused by reactive oxygen species and oxidative stress during in vitro culture, resulting in loss of self-proliferation and multipotency. In this regard, Korean Patent Registration No. 1980562 discloses oxalate as a component for improving stemness of cells. However, despite these efforts, there still is a demand for a technology capable of producing stem cells in which the stemness is maintained even during in vitro culture and augmented.

Sialyloligosaccharides are representative human milk oligosaccharide (HMO) components contained in breast milk, which as a key component of brain components, they play an important role in the development of brain function in infants and are known to have prebiotic efficacy for improving intestinal function. However, nothing about technology of using a sialyloligosaccharide for culturing stem cells or augmenting stemness has been reported.

With this background, the present inventors have confirmed a significant relationship between a sialyloligosaccharide and the stemness of stem cells as a result of making diligent efforts to develop a technology for augmenting stemness of stem cells, thereby completing the present application.

### DESCRIPTION OF EMBODIMENTS

### TECHNICAL PROBLEM

According to an aspect of an embodiment, provided is a composition for augmenting stemness of stem cells, wherein the composition includes a sialyloligosaccharide.

According to another aspect of an embodiment, provided is a method of culturing stem cells, wherein the method includes culturing stem cells in a medium including a sialyloligosaccharide.

According to another aspect of an embodiment, provided is a method of augmenting stemness of stem cells, wherein the method includes adding a sialyloligosaccharide to the stem cells.

According to another aspect of an embodiment, provided is stem cells in which stemness is augmented, wherein the stem cells are obtained using the method of culturing the stem cells.

According to another aspect of an embodiment, provided is a composition for cell therapy, wherein the composition includes the stem cells having augmented stemness and a diluent thereof as an effective ingredient.

According to another aspect of an embodiment, provided is a composition for culturing hepatic organoid, wherein the composition includes a sialyloligosaccharide.

Other objects and advantages of the present disclosure will be apparent by the appended claims and the following detailed description together with the attached drawings. The contents that are not set forth in the specification can be sufficiently recognized and inferred by a person skilled in the art to which the present disclosure belongs or the art similar thereto, and descriptions thereof will be omitted.

### SOLUTION TO PROBLEM

As a result of making diligent efforts to maximize characteristics of stem cells appropriate for a cell therapeutic agent by maintaining stemness and suppressing aging of the stem cells, the present inventors have confirmed that treating stem cells with a sialyloligosaccharide has significantly augmented stemness of the stem cells, as the treatment suppresses senescence of stem cells, increases expression of stem cell factors, and increases cell proliferation ability. Also, they confirmed that treating hepatic organoid with a sialyloligosaccharide may enhance proliferation and/or maturity, thereby completing the present invention based on this.

According to an aspect of an embodiment, provided is a composition for augmenting stemness of stem cells, the composition including a sialyloligosaccharide.

As used herein, the term "stem cells" refers to undifferentiated cells that are capable of infinite proliferation through self-renewability and to cells having pluripotency or multipotency that enable the cells to differentiate into all various types of cells, when an appropriate signal is provided as needed under the influence of the environment in which the cells are located.

Stem cells may be autologous stem cells or allogeneic stem cells, and may be derived from any kind of animal such as a human or a non-human mammal, or from an adult or an embryo, but embodiments are not limited thereto.

Examples of the stem cells may include induced pluripotent stem cells, embryonic stem cells, and adult stem cells, and the stem cells may be, in particular, adult stem cells. The adult stem cells may be mesenchymal stem cells, human tissue-derived mesenchymal stromal cells, human tissue-derived mesenchymal stem cells, multipotent stem cells, or amniotic epithelial cells, but embodiments are not limited thereto. The adult stem cells may be stem cells derived from umbilical cord, umbilical cord blood, synovial membrane, bone marrow, fat, muscle, nerve, skin, amniotic membrane, and placenta, but embodiments are not limited thereto. In one embodiment, the stem cells may be mesenchymal stem cells derived from human bone marrow.

As used herein, the term "induced pluripotent stem cells (iPSCs)" refer to cells induced from differentiated cells by an artificial dedifferentiating process so as to have pluripotent differentiation potential and is also referred to as reprogrammed stem cells. The artificial dedifferentiating process may be performed by the use of a virus-mediated vector such as retrovirus or lentivirus or a nonviral vector or by introduction of nonvirus-mediated dedifferentiating factors using proteins and cell extracts, or includes a dedifferentiating process that is performed by stem cell extracts or compounds. Induced pluripotent stem cells have properties almost similar to those of embryonic stem cells. Particularly, induced pluripotent stem cells show similarity in cell morphology and expression patterns of gene and protein to those of embryonic stem cells, have pluripotency *in vitro* and *in vivo,* form teratomas, and generate chimeric mice upon injection into mouse blastocysts, and are capable of germline transmission of gene. Examples of the induced pluripotent stem cells may include those derived from any animals, including humans, monkeys, pigs, horses, cows, sheep, dogs, cats, mice, and rabbits.

As used herein, the term "embryonic stem cells" refers to pluripotent or totipotent cells that are obtained by in vitro culture of inner cell masses extracted from blastocysts immediately before implantation into the uterus of the mother, are capable of differentiating into all the tissues of the body, and have self-renewal potential with pluripotency or totipotency. In a broad sense, the term also includes embryoid bodies derived from embryonic stem cells. Examples of the embryonic stem cells may include embryonic stem cells derived from humans, monkeys, pigs, horses, cattle, sheep, dogs, cats, mice, or rabbits.

As used herein, the term "adult stem cells" refer to stem cells that are found either in the stage in which individual organs of embryos are formed after developmental or at an adult stage. Adult stem cells may be limited to cells that are differentiated only into cells generally constituting a specific tissue, and such adult stem cells serve to replenish the loss of cells normally or pathologically occurring in most of organs in an adult. The adult stem cells may refer to multipotent cells which is isolated from tissues and cultured *ex vivo.* Examples of the adult stem cells may include adult stem cells derived from humans, monkeys, pigs, horses, cattle, sheep, dogs, cats, mice, or rabbits.

As used herein, the term "mesenchymal stem cells" refers to undifferentiated stem cells isolated from human or mammalian tissues, which may be derived from various tissues. In particular, mesenchymal stem cells may be umbilical cord-derived mesenchymal stem cells, umbilical cord blood-derived mesenchymal stem cells, bone marrow-derived mesenchymal stem cells, adipose-derived mesenchymal stem cells, muscle-derived mesenchymal stem cells, nerve-derived mesenchymal stem cells, skin-derived mesenchymal stem cells, amnion-derived mesenchymal stem cells, and placenta-derived mesenchymal stem cells, and methods for isolating stem cells from respective tissues are well-known in the art.

As used herein, the term "sialyloligosaccharide" refers to an oligosaccharide including at least one sialic acid moiety, and the oligosaccharide is a sugar in which 2 to 8 sugar molecules, such as glucose, fructose, or galactose are bonded, and is a watersoluble crystalline material. Examples of the sialyloligosaccharide may include 3'-sialyllactose, 6'-sialyllactose, 3'-sialyllactosamine, 6'-sialyllactosamine, 3'-sialyl-3-fucosyllactose, sialyllacto-*N*-tetraose, disialyllactose, and a combination thereof, and, for example, the sialyloligosaccharide may be 3'-sialyllactose, which is approved of generally recognized as safe (GRAS) by the US Food and Drug Administration (FDA), but embodiments are not limited thereto.

Also, the composition may further include an oligosaccharide included in human breast milk, i.e., a human milk oligosaccharide. Examples of the human milk oligosaccharide may include lacto-N-tetraose, 2'-fucosyllactose, 3-fucosyllactose, lacto-N-neotetraose, LS-tetrasaccharide b, LS-tetrasaccharide c, disialyllacto-N-tetraose, or a combination thereof, but embodiments are not limited thereto.

Although a sialyloligosaccharide is known as an immune active material having excellent antibacterial and antiviral functions, the functions and activities of a sialyloligosaccharide in relation to stem cell culture, particularly, maintaining and augmenting are not known at all. In one embodiment, a sialyloligosaccharide, e.g., a sialyllactose is found to be closely related to stemness of stem cells. In particular, it was confirmed that mesenchymal stem cell treated with a sialyllactose increases expression of stem cell factors such as SOX2, OCT4, and NANOG, suppresses cell senescence, and improving cell proliferation ability. Thus, a sialyloligosaccharide may be used as an effective ingredient for maintaining or augmenting stemness of stem cells.

As long as the sialyloligosaccharide meets the objective of augmenting stemness of stem cells, the sialyloligosaccharide may be included at an appropriate concentration according to the specific target cells. The sialyloligosaccharide may be included in a concentration in a range of, for example, about 0.1 µM to about 400 µM, for example, about 0.1 µM to about 350 µM, for example, about 0.1 µM to about 200 µM, for example, about 0.1 µM to about 150 µM, for example, about 1 µM to about 400 µM, for example, about 1 µM to about 350 µM, for example, about 1 µM to about 300 µM, for example, about 1 µM to about 250 µM, for example, about 1 µM to about 200 µM, for example, about 1 µM to about 150 µM, for example, about 10 µM to about 250 µM, for example, about 30 µM to about 250 µM, for example, about 50 µM to about 250 µM, for example, about 50 µM to about 200 µM,or, for example, about 50 µM to about 150 µM. When the concentration is higher or lower than these ranges, side effects may be accompanied or maintaining or augmenting stemness of the stem cells by the sialyloligosaccharide may not be exhibited.

As used herein, the term "stemness" is commonly used in a related art as a meaning generally encompassing pluripotency of stem cells to produce all types of cells such as embryonic stem cells and self-renewal capacity of stem cells to unlimitedly produce self-like cells. That is, the stemness may mean ability of maintaining characteristics of stem cells.

As used herein, the term "augmenting stemness" may refer to characteristics that include suppressing cell senescence of stem cells, increasing cell proliferation ability, increasing stem cell functionality, increasing telomerase activity, increasing expression of stem cell factors, proliferating undifferentiated cells while maintaining the undifferentiated state, increasing protein homeostasis, or increasing cell migration activity.

The term "stem cell factors" refers to genes that are much expressed in stem cells and known as serving an important role in maintaining pluripotency, i.e., a characteristic is of stem cells, and examples of the stem cell factors may include SOX2, OCT4, NANOG, KLF4, C-MYC, Oct3/4, SSEA-1, SSEA-3, SSEA-4, TRA1-60, TRA1-81, Lin28, and Fbx15, but embodiments are not limited thereto. Thus, when expression of at least one of the stem cell factors is observed, the cells may be determined as undifferentiated stem cells, and as the expression of the cells increases, it may be determined as the pluripotency of stem cells is high.

The term "increasing functionality" refers to functional maturity and genes known as serving an important role in expressing in stem cells and maintaining mature functional characteristics of the stem cells, where examples of the genes may include CYP3A4, CYP3A7, ALB, AFP, TTR, and CK19, but embodiments are not limited thereto. Thus, when expression of at least one of the factors increasing functionality of stem cells is observed, the cells may be determined capable of increasing functionality of stem cells, and as the expression of the cells increases, it may be determined as the pluripotency of stem cells is high.

The term "proliferation" refers to an increase in the number of cells and may be used as the same meaning of growth. In particular, the term "undifferentiated proliferation" refers that stem cells proliferate into cells having the same properties with the original cells, i.e., cells having multipotency (also referred to "pluripotency") while not being differentiated into specific cells. The composition may be provided in a medium composition form including a sialyloligosaccharide to augment stemness of stem cells. The sialyloligosaccharide may be used as being added in a medium commonly used in the art for culturing stem cells in order to achieve the effect related to augmenting stemness disclosed in the present specification. In one embodiment, it was confirmed that adding a sialyloligosaccharide in a stem cell culturing medium suppresses senescence of stem cells, improves cell proliferation ability, and significantly augmenting stemness.

The medium composition may further include an antioxidant selected from the group consisting of selenium, ascorbic acid, vitamin E, catechin, lycopene, beta-carotene, coenzyme Q-10 (CoQ-10), resveratrol, T-BHQ, oltipraz, alnus japonica extract, eicosapentaenoic acid (EPA), docosahexaenoic acid (DHA), and a combination thereof. The stem cells cultured in the medium composition including the sialyloligosaccharide and the antioxidant may show excellent proliferation ability of cells without change in the shape or activity.

The sialyloligosaccharide; or the sialyloligosaccharide and antioxidant selected from the group consisting of selenium, ascorbic acid, vitamin E, catechin, lycopene, beta-carotene, CoQ-10, resveratrol, T-BHQ, oltipraz, alnus japonica extract, EPA, DHA, and a combination thereof in the medium composition may be used together with a basal medium having a simple composition and known as appropriate for stem cell culturing in the art. Examples of the basal medium commonly used in cell culture may include a basal medium selected from the group consisting of the medium is a basal medium selected from the group consisting of Dulbecco's Modified Eagle's Medium (DMEM), 80 % knockout DMEM, Minimal Essential Medium (MEM), Basal Medium Eagle (BME), RPMI 1640, F-10, F-12, DMEM-F12, α-Minimal Essential Medium (a-MEM), Glasgow's Minimal Essential Medium (G-MEM), Iscove's Modified Dulbecco's Medium (IMDM), MacCoy's 5A medium, AmnioMax Medium, and Chang's Medium MesemCult-XF Medium, and any medium that is used in culturing stem cells in the art may be used.

The medium composition may further include an additional component selected from the group consisting of N-acetyl-L-cysteine (NAC), insulin or insulin-like factor, hydrocortisone, dexamethasone, basic fibroblast growth factor (bFGF), heparan sulfate, 2-mercaptoethanol, epidermal growth factor (EFG), B-27, activin A, BMP-4, oncostatin M (OSM), hepatocyte growth factor (HGF), and a combination thereof. For example, the medium may contain insulin-like factor as insulin replacement, which functions to promote cell growth by enhancing glucose metabolism and protein metabolism. Particularly, recombinant IGF-1 (insulin-like growth factor-1) may be preferably used. A preferred amount of insulin-like factor is in a range of 10 ng/ml to 50 ng/ml. When the amount of insulin-like factor is less than 10 ng/ml, apoptosis will occur, and when the amount is more than 50 ng/ml, the cytotoxicity and cost of the medium may increase. Also, for example, the medium may contain basic fibroblast growth factor (bFGF), which may cause cell proliferation of various types *in vivo,* and a recombinant protein may be preferably used. A preferred amount of bFGF may be in a range of 1 ng/ml to 100 ng/ml. Also, for example, the medium may further include a component selected from the group consisting of fetal bovine serum (FBS), calcium, and epidermal growth factor (EGF), the EGF may be preferably used as a recombinant protein, and a preferable amount of the EGF may be in a range of 10 ng/ml to 50 ng/ml. When the amount of the EGF is less than 10 ng/ml, the medium may have no particular effect, and when the amount of the EGF is more than 50 ng/ml, the medium may be toxic to cells.

The composition may be locally administered to a lesion site. The composition may be locally administered to a lesion site and thus may significantly enhance stemness and cell proliferation ability of stem cells present at or near the lesion site. Also, the composition may be administered together with stem cells cultured in vitro. For local administration, the composition may be combined with, for example, an organic material such as a bio polymer, an inorganic material such as hydroxyapartate, particularly, collagen matrix, polylactic acid or a copolymer thereof, polyethyleneglycol or a copolymer thereof, or a chemical derivative thereof.

The composition may be provided in an injectable form. The composition of an injectable form may be administered systemically or locally by intravenous injection, intramuscular injection, intraperitoneal injection, or subcutaneous injection. In particular, the composition may be provided in an injectable form to facilitate local administration. A sialyloligosaccharide included as an active ingredient in the composition of an injectable form may be dissolved in a pharmaceutically acceptable aqueous solution or may be frozen in a solution state.

The sterile composition for the injection form may be prescribed according to conventional formulation using a vehicle such as distilled water for injection. Examples of the aqueous solution for injection may include physiological saline and isotonic solutions containing glucose or other adjuvants (for example, D-sorbitol, D-mannose, D-mannitol, and sodium chloride). The composition may be used in combination with an appropriate solubilizer, and examples of the solubilizer may include alcohols (e.g., ethanol), polyalcohols (e.g., propylene glycol or polyethylene glycol), and non-ionic surfactants (e.g., Polysorbate 80 (registered trademark) or HCO-50). Oily liquids include sesame oil and soybean oil, and they may be used in combination with benzyl benzoate and/or benzyl alcohol as solubilizers. The composition may be used in combination with buffers (e.g., phosphate buffer and sodium acetate buffer), analgesics (e.g., procaine hydrochloride), stabilizers (e.g., benzyl alcohol and phenol), and/or antioxidants. The prepared injections may be filled into generally appropriate ampules.

According to another aspect of an embodiment, provided is a method of culturing stem cells, the method including culturing stem cells in a medium including a sialyloligosaccharide.

In the method of culturing stem cells, descriptions of the "sialyloligosaccharide" and "stem cells" are the same as those defined above.

As used herein, the term "medium" means a medium capable of supporting the growth and survival of stem cells under in vitro culture conditions, and is intended to include all conventional media known in the art to be suitable for the culture of stem cells. A medium and culture conditions may be selected depending on the type of cells to be cultured. The medium used in the culturing is a cell culture minimum medium (CCMM), which generally includes a carbon source, a nitrogen source, and trace elements.

Examples of the CCMM include, but are not limited to, Dulbecco's Modified Eagle's Medium (DMEM), Endothelial differentiation medium (EDM), Minimal Essential Medium (MEM), Basal Medium Eagle (BME), RPMI 1640, F-10, F-12, α-Minimal Essential Medium (a-MEM), Glasgow's Minimal Essential Medium (G-MEM), or Iscove's Modified Dulbecco's Medium (IMDM). In the CCMM, an antibiotic, such as penicillin, streptomycin, and gentamicin, may be added.

The medium may include a sialyloligosaccharide at an effective concentration. The term "effective concentration" means an amount of a sialyloligosaccharide sufficient to augment stemness of stem cells. Since a sialyloligosaccharide does not show activity under the effective concentration, and may have toxicity to cells above the effective concentration, a sialyloligosaccharide may be used within the effective concentration. The sialyloligosaccharide may be included in a concentration in a range of, for example, about 0.1 µM to about 400 µM, for example, about 0.1 µM to about 350 µM, for example, about 0.1 µM to about 300 µM, for example, about 0.1 µM to about 250 µM, for example, about 0.1 µM to about 200 µM, for example, about 0.1 µM to about 150 µM, for example, about 1 µM to about 400 µM, for example, about 1 µM to about 350 µM, for example, about 1 |jM to about 300 µM, for example, about 1 µM to about 250 µM, for example, about 1 µM to about 200 µM, for example, about 1 µM to about 150 µM, for example, about 10 µM to about 250 µM, for example, about 30 µM to about 250 µM, for example, about 50 µM to about 250 µM, for example, about 50 µM to about 200 µM,or, for example, about 50 µM to about 150 µM. The stem cell maintaining or promoting efficacy may not appear at a concentration in these concentration ranges.

Also, the medium may further include a human milk oligosaccharide. The human milk oligosaccharide may include, for example, lacto-N-tetraose, 2'-fucosyllactose, 3-fucosyllactose, lacto-N-neotetraose, LS-tetrasaccharide b, LS-tetrasaccharide c, disialyllacto-N-tetraose, or a combination thereof, but embodiments are not limited thereto.

The method may include subculturing stem cells. The method may maintain the stem cells in an undifferentiated state and, particularly, may maintain the stem cells in an undifferentiated state during or even after the subculturing. For example, the method may maintain the stemness after subculturing the stem cells of 3 to 10 or more passages.

As used herein, the term "subculture" means that a portion of cells is periodically passaged by transferring them to a new culture vessel and by replacing the culture medium with a fresh culture for the long-term culture of healthy cells. The term "passage" is defined herein as the growth of human pluripotent stem cells from an initial seed culture in a culture vessel to confluence in the same culture vessel. As the number of cells increases in the limited space of the culture vessel, the cells die naturally due to nutrient depletion or waste accumulation in a predetermined time. Thus, subculture is used to increase the number of healthy cells. Typically, 1 passage means culture by one replacement of medium (culture vessel) or one isolation of cell population. Any subculture method known in the art may be used without any limitation, but a mechanical or enzymatic isolation method may be preferably performed.

Stem cells have problems in that, when the cells are repeatedly cultured or receive external stimuli, the morphology or size of the cells is minutely changed and the self-renewal ability and telomerase activity of the cells are reduced, that is, the cells enter senescence. Because the self-renewal ability and telomerase activity of cells differ between media in which the cells are cultured, it is important to culture stem cells in a medium composition which does not reduce the self-renewal ability or telomerase activity of the cells without significantly changing the characteristics of the cells even when the cells are repeatedly cultured. In other words, it is important to culture stem cells in a medium composition which delays or alleviates the senescence of the cells or maintains the cells in the initial state. However, in the case in which stem cells are cultured in conventional medium compositions, the stem cells should be subcultured several times in order to increase the yield of the stem cells, and thus large amounts of manpower and time are required. Particularly, in this case, some of the medium components required for subculture are very costly, and thus the conventional medium compositions are disadvantageous in economic terms. In addition, there is a problem in that the self-renewal ability of the cells is reduced due to repeated subculture. To overcome these problems, the medium composition according to an embodiment provides a medium composition capable of enhancing the self-renewal ability of cells without changing the morphology or activity of the cells. Thus, in one aspect, provided is a medium composition including a sialyloligosaccharide as an active ingredient for maintaining and augmenting stemness.

The maintaining of an undifferentiated state of the stem cells may be confirmed by an increase in expression of genes such as alkaline phosphatase (ALP), OCT-4, SOX2, human telomerase reverse transcriptase (hTERT), teratocarcinoma-derived growth factor (TDGF), or SSEA-4, but embodiments are not limited thereto.

In one embodiment, it was confirmed that senescence of the mesenchymal stem cells not treated with a sialyllactose after the subculturing was in progress as the expressions of p16 and p53, which are cell senescence factors, increased, whereas it was confirmed that senescence of the mesenchymal stem cells treated with a sialyllactose was suppressed as the expressions of p16 and p53 were significantly reduced.

Also, in one embodiment, it was confirmed that senescence of the mesenchymal stem cells not treated with a sialyllactose after the subculturing was in progress as the activity of SA-β-gal, which is a cell senescence marker, increased, whereas it was confirmed that senescence of the mesenchymal stem cells treated with a sialyllactose was suppressed as the activity of SA-β-gal was significantly reduced.

It is to be understood that among the terms or elements mentioned in the method of culturing stem cells, those mentioned in the description of the composition for augmenting stemness are the same as mentioned in the description of the composition above.

Still another aspect provides a method of augmenting stemness of stem cells, the method including adding a sialyloligosaccharide to stem cells.

In the method of augmenting stemness of stem cells, the terms such as "sialyloligosaccharide" and "stem cells" are the same as described above.

The adding of the sialyloligosaccharide to stem cells may be performed *ex vivo* or *in vitro.* Also, the adding of the sialyloligosaccharide may refer to adding a sialyloligosaccharide to stem cells that is being cultured in a medium.

The adding of the sialyloligosaccharide may be performed in normal serum medium or may be performed after transferring the stem cells subcultured in serum medium into serum-free medium. The cultivation in serum-free medium may be performed after removing the culture of stem cells cultured in serum medium followed by washing the cells with phosphate buffer.

The method may further include adding a human milk oligosaccharide. The adding of the human milk oligosaccharide may be performed simultaneously, separately, or sequentially performed with the adding of the sialyloligosaccharide, and examples of the human milk oligosaccharide may include lacto-N-tetraose, 2'-fucosyllactose, 3-fucosyllactose, lacto-N-neotetraose, LS-tetrasaccharide b, LS-tetrasaccharide c, disialyllacto-N-tetraose, or a combination thereof, but embodiments are not limited thereto.

The term "a serum-free medium" refers to a serum-free culture solution and medium for culturing animal tissues or cells. For example, the serum-free medium may be Dulbecco's Modified Eagle's Medium (DMEM), Endothelial differentiation medium (EDM), Minimal Essential Medium (MEM), Basal Medium Eagle (BME), RPMI 1640, F-10, F-12, α-Minimal Essential Medium (a-MEM), Glasgow's Minimal Essential Medium (G-MEM), or Iscove's Modified Dulbecco's Medium (IMDM).

The term "serum" refers to a clear supernatant that is isolated from blood after the blood has fully coagulated. Also, serum must be added to the synthetic medium to culture animal cells, where cow, horse, or human serum may be used, and cow serum is most commonly used. Depending on when blood is collected, serum may be divided into fetal bovine serum (FBS), newborn bovine serum, calf serum, and bovine serum. Examples of the serum may include fetal bovine serum (FBS) and bovine calf serum (BCS).

According to the method of augmenting stemness of stem cells, stem cells having characteristics of stem cells appropriate as a cellular therapeutic agent as treating stem cells with a sialyloligosaccharide suppresses senescence of stem cells and significantly increases cell proliferation ability, that is, significantly augments stemness of the stem cells *in vitro* and *in vivo.*

It is to be understood that among the terms or elements mentioned in the method of augmenting stemness of stem cells, those mentioned in the description of the composition for augmenting stemness or the method of culturing stem cells are the same as mentioned in the description of the composition or the method above.

According to another aspect of an embodiment, provided are stem cells having augmented stemness obtained using the method of augmenting stemness.

According to another aspect of an embodiment, provided is a cellular therapeutic composition including the stem cells having augmented stemness or a dilution thereof.

According to the treating the stem cells with a sialyloligosaccharide, stemness of the stem cells may be augmented. Therefore, the stem cells obtained using the method of augmenting stemness, the method comprising treating the stem cells with a sialyloligosaccharide, may have significantly augmented stemness, and thus the stem cells may be included as an active ingredient of a cellular therapeutic agent by activating the stem cells into a state appropriate for transplantation or change their characteristics suitable to human body.

As used herein, the term "cellular therapeutic agent" refers to a drug used for the purpose of treatment, diagnosis, and prevention, which contains a cell or tissue prepared through isolation from humans, culture and specific operation. Particularly, it refers to a drug used for the purpose of treatment, diagnosis, and prevention through a series of behaviors of in vitro multiplying and sorting living autologous, allogenic and xenogenic cells or changing the biological characteristics of cells by other means for the purpose of restoring the functions of cells and tissues. Cellular therapeutic agents are broadly divided, according to the differentiation level of cells, into somatic cell therapeutic agents and stem cell therapeutic agents. In one embodiment, the cellular therapeutic agent composition may be a stem cell therapeutic agent including stem cells treated with a sialyloligosaccharide as an active ingredient.

As used herein, the term "stem cell therapeutic agent" is a type of cell therapeutic agent that manipulates cells *ex vivo* for the purpose of treating, diagnosing, and preventing diseases, and the agent is injected back into the human body. It refers to a customized biopharmaceutical drug that fundamentally treats diseases by injecting manipulated and cultured stem cells into patients, unlike conventional medicines aimed at alleviating symptoms of a disease for an unspecified number of people. Depending on the origin of the cells, the stem cell therapeutic agent may be classified into an embryonic stem cell therapeutic agent that is produced at an early stage of development of an embryo, an adult stem cell therapeutic agent that exists limitedly in various tissues of adults, a dedifferentiation stem cell therapeutic agent that is induced to an initial undifferentiated state by performing dedifferentiation on normal somatic cells, and an organoid-based cell therapeutic agent derived from dedifferentiated stem cells. Also, depending on the treatment method, the stem cell therapeutic agent may be classified into a self stem cell therapeutic agent that transplants stem cells of the patient himself, an allogeneic stem cell therapeutic agent that transplants stem cells of other humans, and a heterogeneous stem cell therapeutic agent using animal stem cells. Since the stem cell therapeutic agent may recover the function or tissue of damaged cells by directly injecting cells into the patient, a fundamental treatment method not toxic to the human body may be suggested as the treatment regenerates and maintains the original function of the body which may not be done by surgery or drug therapy. The stem cell therapeutic agent may be utilized in treatment of various cell damage diseases such as cerebral infarction, myocardial infarction, degenerative arthritis, and bone fractures through the 3R strategy of Regeneration and rejuvenation, Replacement, and Repair of damaged tissues and cells.

The stem cells with augmented stemness may be preferably in the form of a cell composition including one or more diluents to protect and maintain the cells, and to facilitate the use of the stem cells during injection or transplantation into a target tissue. Examples of the diluent may include a buffer solution such as physiological saline, phosphate buffered saline (PBS), and a Hank's balanced salt solution (HBSS); plasma; or blood components.

The cellular therapeutic agent composition may be administered into mammals including humans through various routes. The administration method may be all the commonly used methods, and for example, it may be administered through parenteral administration in the form of intramuscular or intravenous injections, as well as direct administration to the diseased site, and it may be administered through oral, percutaneous, subcutaneous, intravenous, or intramuscular routes.

The dosage of the cellular therapeutic agent composition may differ according to the age, body weight, gender, administration form, health condition, and disease degree of patients, and it may be administered as divided once to several times a day at regular intervals, depending on the decision of a doctor or pharmacist. For example, in case of humans, the common dose of the cellular therapeutic agent may be 1.0 x 10⁴ to 1.0 x 10¹⁰ cells/kg body weight, preferably, 1.0 x 10⁶ to 1.0 x 10⁸ cells/kg body weight, which may be administered as divided into once to several times. The dose is illustrative of the average case, and may be high or low depending on individual differences.

The cell therapeutic agent composition may exhibit preventive or therapeutic effect on neurological diseases, muscular system diseases, bone diseases, or epithelial-related diseases. The neurological diseases may include degenerative neurological diseases, and may include a variety of neurological diseases, for example, Parkinson's disease, dementia, Alzheimer's disease, Huntington's disease, amyotrophic axonal sclerosis, stroke, memory loss, myasthenia gravis, progressive supranuclear palsy, multiple system atrophy, essential tremor, cortical-basal ganglia degeneration, diffuse Lewy body disease, and Pick's disease. The muscular diseases may include muscular atrophy, cerebral palsy, progressive ossified fibrodysplasia, dermatomyositis, compartment syndrome, myasthenia gravis, Lou Gehrig's disease, mitochondrial myopathy, rhabdomyolysis, polymyositis, fibromyalgia, myotonia, alcoholic hepatitis, nonalcoholic fatty liver, hepatitis, cirrhosis, and myofascial pain syndrome. The bone diseases may include osteoporosis, osteomalacia, osteopenia, bone atrophy, osteoarthritis, avascular necrosis of the Femoral Head, or periodontal disease. The epithelial-related diseases may include an intractable corneal disease. The cellular therapeutic agent composition may be applied to all diseases that may be treated by transplanting stem cells in addition to these diseases.

As used herein, the term "treatment" used herein refers to all actions involved in preventing, alleviating or beneficially changing clinical situations related to a disease. In addition, the treatment may refer to an increased survival compared with an expected survival rate when untreated. The treatment may include a preventive means in addition to a therapeutic means.

The cellular therapeutic agent composition may be formulated by further including a pharmaceutically acceptable carrier. As used herein, the term "pharmaceutically acceptable carrier" refers to a carrier or diluent which does not significantly irritate an organism and does not interfere with the biological activity and properties of an administered ingredient. A pharmaceutically acceptable carrier for a pharmaceutical composition, to which the cellular therapeutic agent composition may be applied, may be any one known in the art such as a buffer, a preservative, an analgesic, a solubilizer, an isotonic agent, a stabilizer, a base, an excipient or a lubricant without limitation.

The cellular therapeutic agent composition may be prepared in various formulations according to a technique commonly used. The cellular therapeutic agent composition may be administered by any route as long as it may induce transmission to a disease lesion. In some cases, a method for loading the composition into a vehicle, which includes a means for directing stem cells to a lesion, may be considered. Therefore, the cellular therapeutic agent composition may be administered by various routes including local administration (including buccal, sublingual, skin and intraocular administration), non-oral administration (including subcutaneous, intradermal, intramuscular, infusion, intravenous, intraarterial, intraarticular and intrathecal administration), and transdermal administration, and preferably, it is directly administered to a disease lesion.

In an aspect, stem cells may be suspended in a suitable diluting agent and administered into a subject, and the diluting agent is used to protect and maintain cells, and to facilitate use in injection into desired tissue. The diluting agent may be a buffer solution such as a physiological saline, a phosphate buffered solution, or HBSS, or a cerebrospinal fluid. In addition, the cellular therapeutic agent composition may be administered by a random device to transmit an active ingredient to target cells. A preferable administration method and type of preparation is an injection. The injection may be prepared using an aqueous solvent such as a physiological saline, a Ringer's solution, a Hank's solution or a sterilized aqueous solution, or a non-aqueous solvent such as a vegetable oil (e.g., olive oil), a higherfatty acid ester (e.g., ethyl oleate), ethanol, benzyl alcohol, propylene glycol, polyethylene glycol or glycerin, and for mucosal permeation, a non-permeable agent known in the art and suitable for a barrier to be permeated may be used, and ascorbic acid, sodium hydrogen sulfite, BHA, tocopherol or EDTA may be used as a stabilizer for preventing spoilage, and a pharmaceutical carrier such as an emulsifier, a buffer for pH adjustment, or a preservative for preventing microbial development such as mercury nitrate, thimerosal, benzalkonium chloride, phenol, cresol or benzyl alcohol may be additionally included.

The term "administration" used herein means the introduction of the cellular therapeutic agent composition to a patient by any suitable method, and an administration route of the cellular therapeutic agent composition may vary as long as the composition may reach desired tissue, and it may be any one of various routes including oral and parenteral routes. The composition may be administered intraperitoneally, intravenously, intramuscularly, subcutaneously, intradermally, orally, locally, intranasally, intrapulmonary or intrarectally, but embodiments are not limited thereto.

The cellular therapeutic agent composition may be administered in an effective amount. As used herein, the term "effective amount" refers to a desired amount required to delay or completely interrupt the onset or progression of a certain disease to be treated. A suitable total amount of administration per day may be determined by appropriate medical decisions. The specific therapeutically effective amount of the cellular therapeutic agent composition administered to a particular patient may be varied depending on the type and degree of the response to be achieved in the treatment, the specific composition, including whether another agent is included in the composition, the patient's age, body weight, general health status, sex, diet, administration time, administration route, the ratio of composition, treatment period, other drugs used together in the treatment and a variety of factors well known in the medical field.

It is to be understood that among the terms or elements mentioned in the stem cells or the cellular therapeutic agent composition including the stem cells, those mentioned in the description of the composition for augmenting stemness or the method of culturing stem cells are the same as mentioned in the description of the composition or the culturing method above.

In another aspect, provided is a composition for culturing hepatic organoids, the composition including a sialyloligosaccharide.

In another aspect, provided is a method of preparing matured hepatic organoids/a method of enhancing maturity of hepatic organoids, the method including adding a sialyloligosaccharide to hepatic organoids.

It is to be understood that among the terms or elements mentioned in the composition for culturing hepatic organoids or the method of preparing matured hepatic organoids/the method of enhancing maturity of hepatic organoids, those mentioned in the description of the composition for augmenting stemness or the method of culturing stem cells are the same as mentioned in the description of the composition and the culturing method above.

In one embodiment, it was confirmed that treating a sialyloligosaccharide during a process of culturing the prepared hepatic organoids significantly increases proliferation of hepatic organoids, and that the treatment increased the CYP3A4/CYP3A7 ratios and ALB/AFP ratios, and this confirmed the influenced on maturity of hepatic organoids.

The term "organoid" refers to a cell aggregate made by aggregating and recombining cells isolated from stem cells or organ-derived cells by 3D culture, and may include organoids or cell clusters formed from suspension cell culture. The organoid may also be referred to as a small pseudo-organ, an organ analogue, or a pseudo-organ. Particularly, the organoid includes one or more cell types among various types of cells forming an organ or tissue, and should be able to reproduce the shape and function of the tissue or organ.

The term "organoid culture" includes all actions for producing or maintaining an organoid. For example, it may be differentiating stem cells or cells isolated from particular tissues into tissue or organ cells with specific functions, and/or may be surviving, growing or proliferating an organoid.

The organoid in which the composition may be used may be, for example, an organoid derived from induced pluripotent stem cells, embryonic stem cells, or adult stem cells, or may be preferably a hepatic organoid derived from induced pluripotent stem cells or adult stem cells.

The sialyloligosaccharide may be included in an appropriate concentration as long as the concentration meets the purpose of culturing hepatic organoids or enhancing the maturity. The sialyloligosaccharide may be included in a concentration in a range of, for example, about 0.1 µM to about 400 µM, for example, about 0.1 µM to about 350 µM, for example, about 0.1 µM to about 300 µM, for example, about 0.1 µM to about 250 µM, for example, about 0.1 µM to about 200 µM, for example, about 0.1 µM to about 150 µM, for example, about 1 µM to about 400 µM, for example, about 1 µM to about 350 µM, for example, about 1 µM to about 300 µM, for example, about 1 µM to about 250 µM, for example, about 1 µM to about 200 µM, for example, about 1 µM to about 150 µM, for example, about 10 µM to about 250 µM, for example, about 30 µM to about 250 µM, for example, about 50 µM to about 250 µM, for example, about 50 µM to about 200 µM,or, for example, about 50 µM to about 150 µM.

### ADVANTAGEOUS EFFECTS OF DISCLOSURE

When a composition or a method according to an aspect of an embodiment is used, expression of stem cell factor may significantly increase, senescence of stem cells may be suppressed, and cell proliferation ability of stem cells or cell functionality of stem cells may significantly increase.

The composition or method according to an aspect may resolve the problem of losing self-renewal and multipotency, which is pointed out at in vitro stem cell culture of the related art, and may improve therapeutic efficacy of a cellular therapeutic agent by using stem cells having augmented stemness.

Stem cells prepared using the composition or method according to an aspect may have significantly augmented stemness and thus may be included as an active ingredient of a cellular therapeutic agent.

When the cellular therapeutic agent composition according to an aspect includes stem cells having significantly augmented stemness, the efficacy of the cellular therapeutic agent may increase.

### BRIEF DESCRIPTION OF DRAWINGS

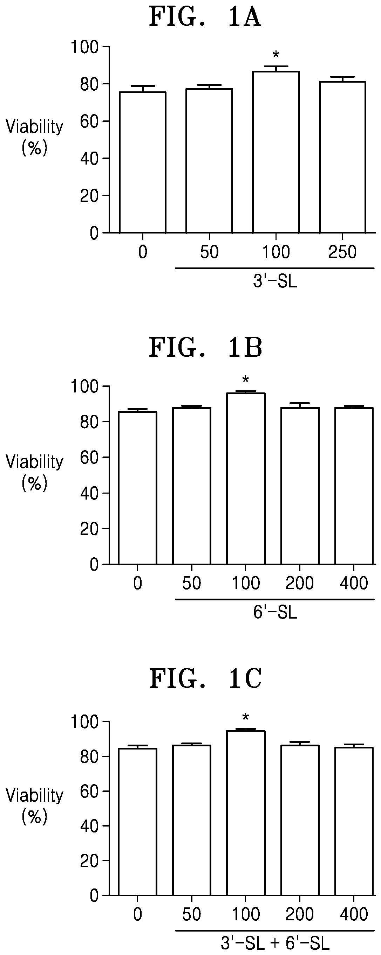

FIG. 1 shows the results of confirming influence of a sialyllactose on cell viability of bone marrow-derived mesenchymal stem cells according to concentrations of a sialyllactose by CCK-8 assay, wherein FIG. 1A shows the results of treating the stem cells with 3'-sialyllactose, FIG. 1B shows the results of treating the stem cells with 6'-sialyllactose, and FIG. 1C shows the results of treating the stem cells with a mixture of 3'-sialyllactose and 6'-sialyllactose;

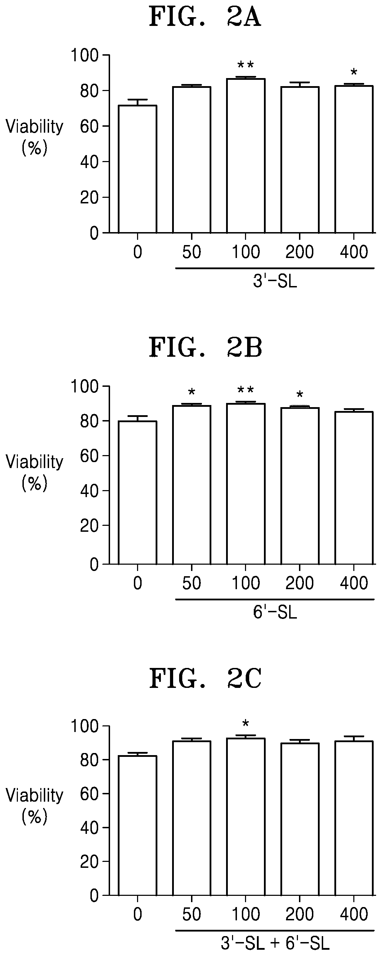

FIG. 2 shows the results of confirming influence of a sialyllactose on cell viability of adipose-derived mesenchymal stem cells according to concentrations of a sialyllactose by CCK-8 assay, wherein FIG. 2A shows the results of treating the stem cells with 3'-sialyllactose, FIG. 2B shows the results of treating the stem cells with 6'-sialyllactose, and FIG. 2C shows the results of treating the stem cells with a mixture of 3'-sialyllactose and 6'-sialyllactose;

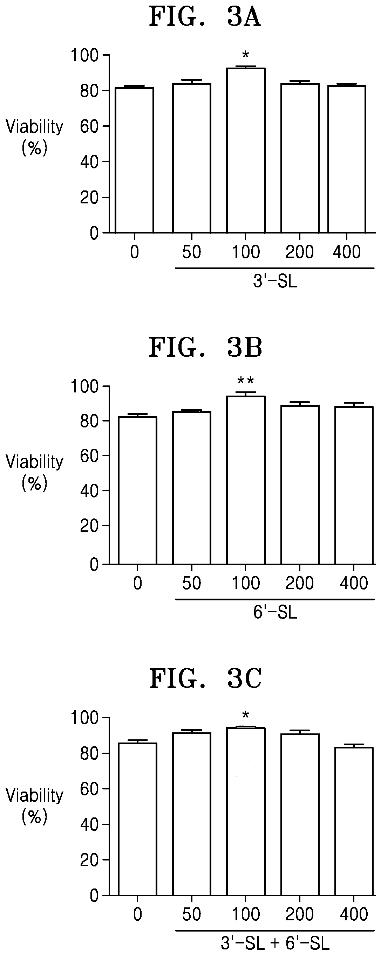

FIG. 3 shows the results of confirming influence of a sialyllactose on cell viability of umbilical cord blood-derived mesenchymal stem cells according to concentrations of a sialyllactose by CCK-8 assay, wherein FIG. 3A shows the results of treating the stem cells with 3'-sialyllactose, FIG. 3B shows the results of treating the stem cells with 6'-sialyllactose, and FIG. 3C shows the results of treating the stem cells with a mixture of 3'-sialyllactose and 6'-sialyllactose;

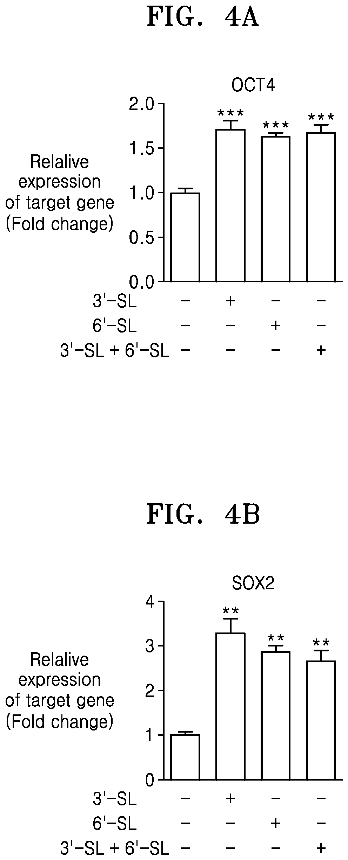

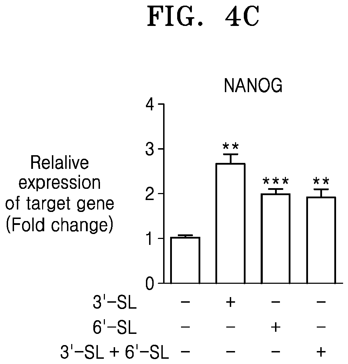

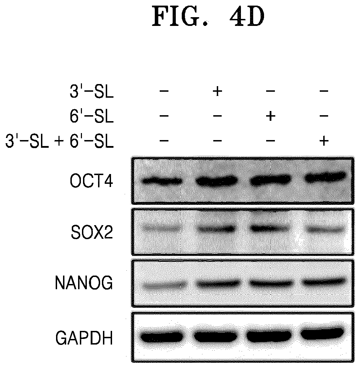

FIG. 4 shows the results of confirming influence of a sialyllactose on stemness of bone marrow-derived mesenchymal stem cells, wherein FIG. 4A shows the results of confirming mRNA levels of OCT4, FIG. 4B shows the results of confirming mRNA levels of SOX2, FIG. 4C shows the results of confirming mRNA levels of NANOG, and FIG. 4D shows the results of confirming protein expression levels of OCT4, SOX2, and NANOG;

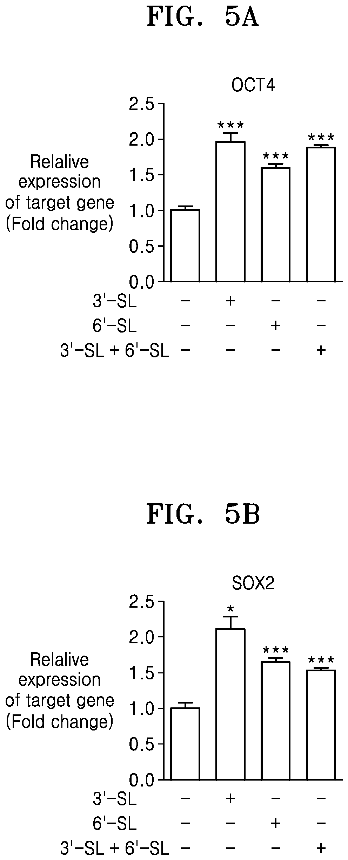

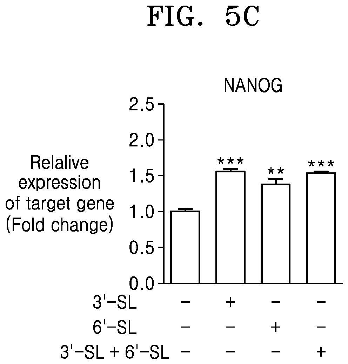

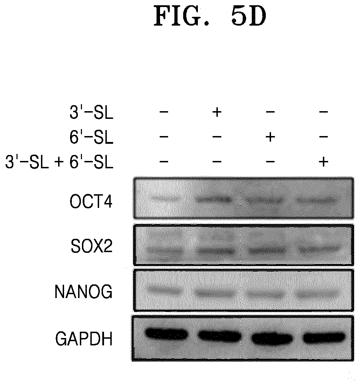

FIG. 5 shows the results of confirming influence of a sialyllactose on stemness of adipose-derived mesenchymal stem cells, wherein FIG. 5A shows the results of confirming mRNA levels of OCT4, FIG. 5B shows the results of confirming mRNA levels of SOX2, FIG. 5C shows the results of confirming mRNA levels of NANOG, and FIG. 5D shows the results of confirming protein expression levels of OCT4, SOX2, and NANOG;

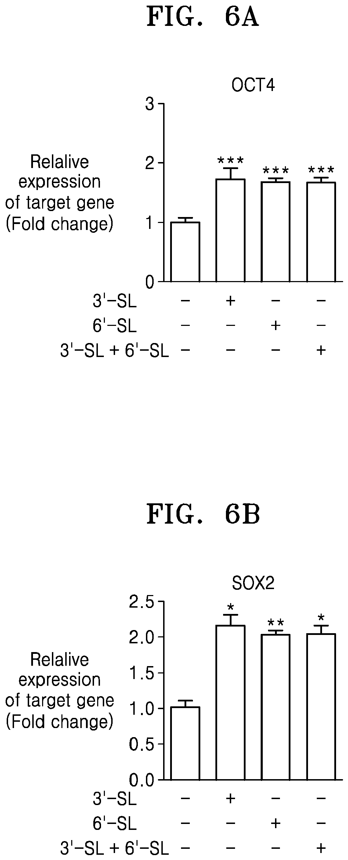

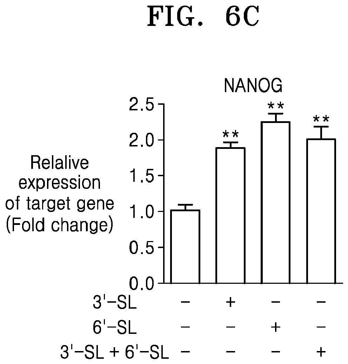

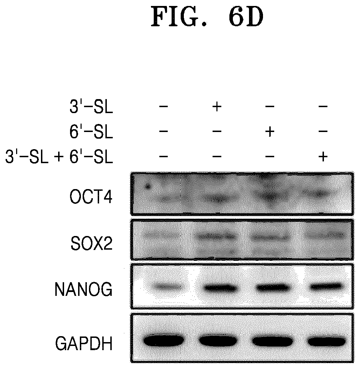

FIG. 6 shows the results of confirming influence of a sialyllactose on stemness of umbilical cord blood-derived mesenchymal stem cells, wherein FIG. 6A shows the results of confirming mRNA levels of OCT4, FIG. 6B shows the results of confirming mRNA levels of SOX2, FIG. 6C shows the results of confirming mRNA levels of NANOG, and FIG. 6D shows the results of confirming protein expression levels of OCT4, SOX2, and NANOG;

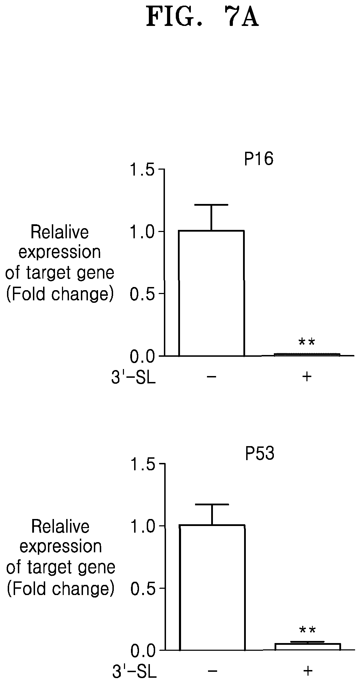

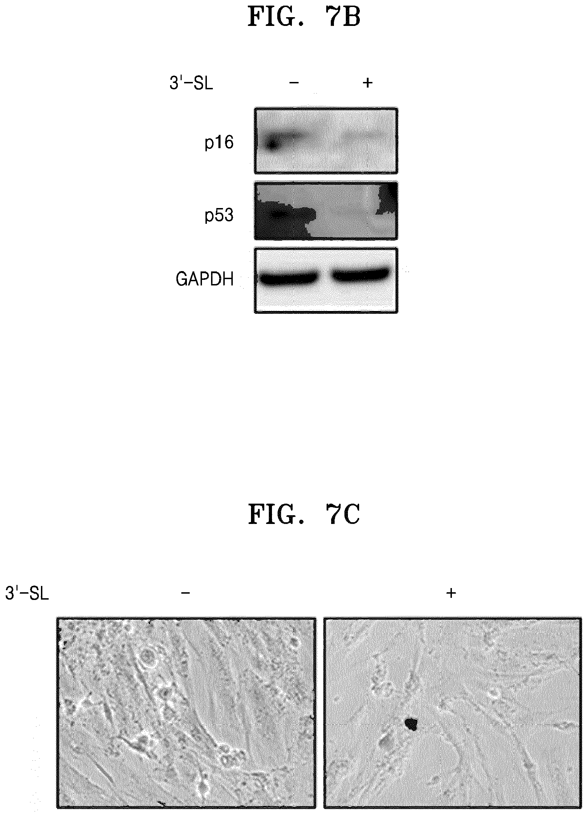

FIG. 7 shows the results of confirming influence of 3'-sialyllactose on senescence of bone marrow-derived mesenchymal stem cells, wherein FIG. 7A shows the results of confirming changes in expressions at mRNA levels of p16 and p53, which are cell senescence factors, FIG. 7B shows the results of confirming changes in expressions at protein levels of p16 and p53, and FIG. 7C shows the results of analyzing cell senescence using SA-β-gal;

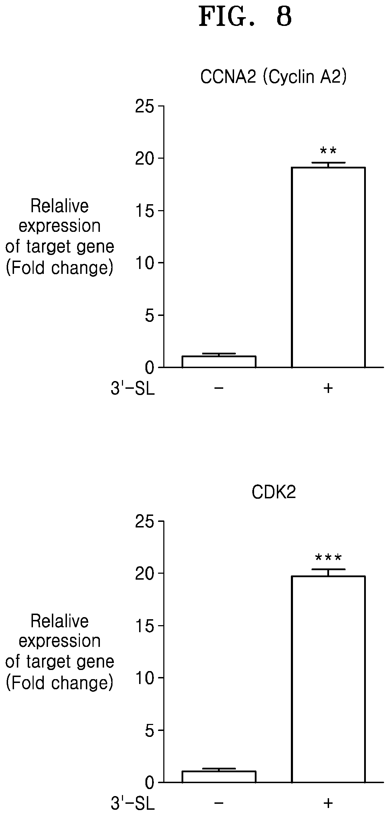

FIG. 8 shows the results of confirming influence of 3'-sialyllactose on proliferation of bone marrow-derived mesenchymal stem cells, and these are the results of confirming changes in expressions of CCNA2 and CDK2, which are genes involved in cell cycle progression and proliferation;

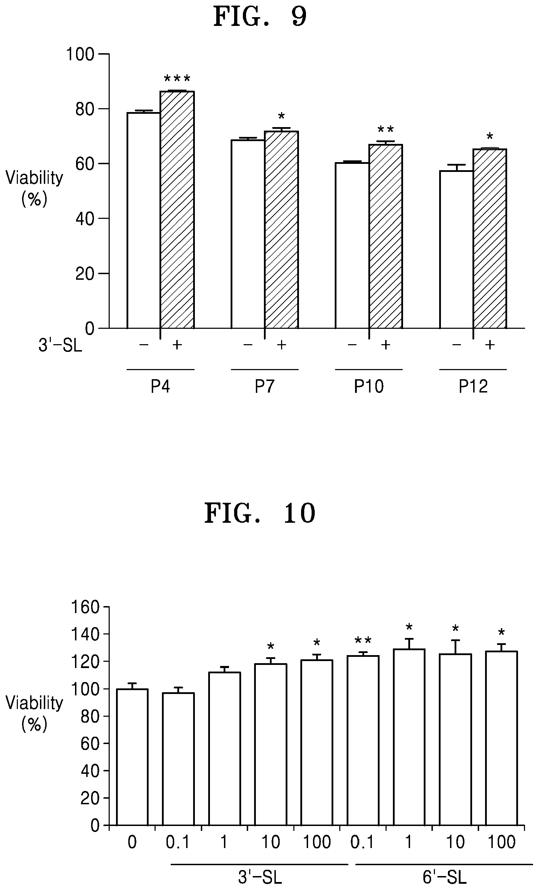

FIG. 9 shows the results of confirming influence of 3'-sialyllactose on cell viability of bone marrow-derived mesenchymal stem cells per subculturing passage;
FIG. 10 shows the results of confirming influence of a sialyllactose on cell viability of induced pluripotent stem cell-derived hepatic organoid cells, and these are the results of confirming the cell viability by CCK-8 assay;

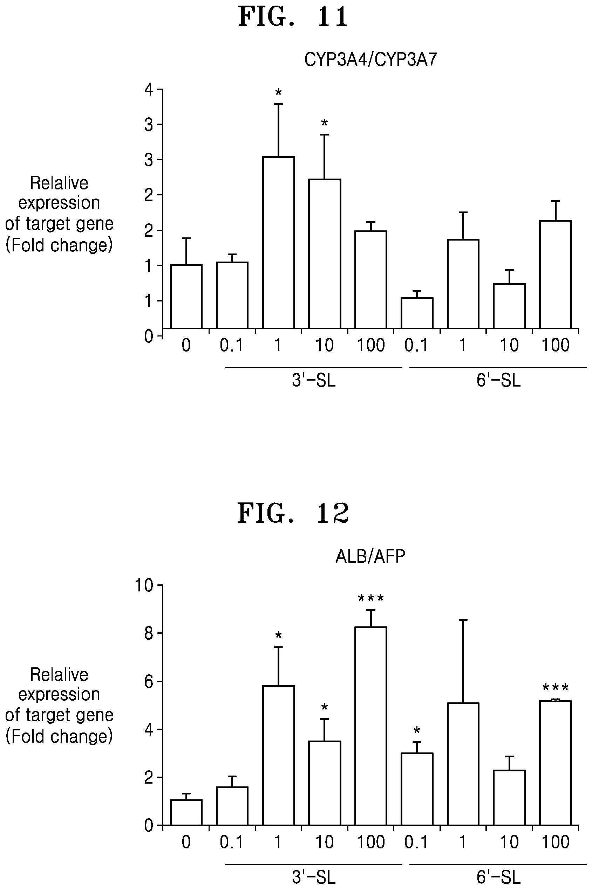

FIG. 11 shows the results of confirming influence of a sialyllactose on maturity of induced pluripotent stem cell-derived hepatic organoid cells, and these are the results of comparing ratios of expressions of CYP3A4 and CYP3A7; and
FIG. 12 shows the results of confirming influence of a sialyllactose on maturity of induced pluripotent stem cell-derived hepatic organoid cells, and these are the results of comparing ratios of expressions of ALB and AFP.

### MODE OF DISCLOSURE

Hereinafter, the present disclosure will now be described in greater detail with reference to the accompanying Examples below. However, these Examples are for illustrative purposes only, and should not be construed as being limited to the scope of the inventive concept.

### Example 1. Culturing stem cells

In Example 1, human bone marrow-derived mesenchymal stem cells (hBMSCs), human adipose tissue-derived mesenchymal stem cells (hATMSCs), human cord blood-derived mesenchymal stem cells (hCBMSCs), and induced pluripotent stem cells-derived hepatic organoid were used in this experiment. Dulbecco's Modified Eagle's Mediums (DMEM Low Glucose, available from HyClone in Logan, UT, USA) including 10 % fetal bovine serum (available from T&I in Gangwon, Korea), 1 % antibiotics (penicillin/streptomycin, available from Gibco in Grandlsland, NY, USA) were used as culture mediums of hBMSCs, hATMSCs, and hCBMSCs. After treating 3'-sialyllactose (available from GeneChem), 6'-sialyllactose (available from GeneChem), and a mixture of 3'-sialyllactose and 6'-sialyllactose, each in an amount of 100 µM, the culturing solutions were replaced every 3 days. The negative control was performed in the same manner after adding triple distilled water into the culturing solution. Also, a Hepatocyte Culture Medium (available from Lonza) including an endothelial cell growth medium-2 (available from Lonza), 2.5 % FBS, 100 nM dexamethasone (available from Sigma-Aldrich), 20 ng/ml OSM (available from R&D system), and 10 ng/ml HGF (available from PeproTech) was used as the culturing solution of hepatic organoid, and the culturing solution was replaced every 3 days.

### Example 2. Confirmation of cell viability according to sialyloligosaccharide treatment

In Example 2, influence of a sialyloligosaccharide on cell viability of stem cells was to be confirmed. Particularly, 3'-sialyllactose, 6'-sialyllactose, and a combination thereof were each treated on the subcultured stem cells in a concentration of 0, 50, 100, 200, or 400 µM, and the cell viability per concentration was verified by cell counting kit-8 (CCK-8) analysis. In particular, of hBMSCs, hATMSCs, and hCBMSCs were seeded in a 96-well plate. Then, the stem cells were treated with 3'-sialyllactose (GeneChem), 6'-sialyllactose (GeneChem), and a mixture of 3'-sialyllactose and 6'-sialyllactose in a concentration of 0, 50, 100, 200, or 400 µM and cultured in a DMEM low glucose medium (HyClone) including 1 % antibiotics free of fetal bovine serum (T&I). Next, the viabilities of the cultured cells were confirmed by CCK-8 (Dojindo Laboratories, Kumamoto, Japan) analysis.

FIGS. 1 to 3 show the results of confirming influence of each of the stem cells on the cell viability after treating 3'-sialyllactose, 6'-sialyllactose, and a mixture of 3'-sialyllactose and 6'-sialyllactose of each concentration in hBMSCs (FIG. 1), hATMSCs (FIG. 2), and hCBMSCs (FIG. 3). As a result, the stem cells did not negatively influence the cell viability in all the concentrations, but when the stem cells were treated with a sialyllactose in a concentration of 100 µM, it was confirmed that the cell viability increased significantly. Therefore, a sialyllactose concentration of the treatment was 100 µM in the following experiments.

### Example 3. Confirmation of stemness according to sialyloligosaccharide treatment

In Example 3, influence of a sialyloligosaccharide on stemness of stem cells was to be confirmed. Particularly, after treating mesenchymal stem cells with each of 3'-sialyllactose, 6'-sialyllactose, and a combination thereof, mRNAs of OCT4, SOX2, and NANOG, which are stem cell markers of the mesenchymal stem cells, and changes in expression at protein levels were each measured by quantitative real-time reverse transcription-polymerase chain reaction (qRT-PCR) and western blot. In particular, total RNAs were isolated from the cells collected after culturing the cells in the same manner as in Example 1, and change in mRNA expression of the isolated total RNAs was confirmed by qRT-PCR using OCT4 primers available from Bioneer (Daejeon, Korea), SOX2 primers available from Bioneer (P200205), and NANOG primers. Sequences of the used OCT4 and NANOG primers are shown in Table 1.

**[Table 1]**

| Primer Name | Primer Sequence (5'->3') | SEQ ID NO: |
|---|---|---|
| OCT4 forward | GCAAGCCCTCATTTCACCA | 1 |
| OCT4 reverse | GCCCATCACCTCCACCAC | 2 |
| NANOG forward | TTTGTGGGCCTGAAGAAAACT | 3 |
| NANOG reverse | AGGGCTGTCCTGAATAAGCAG | 4 |

FIGS. 4 to 6 show the results confirming influence of a sialyllactose on stemness of stem cells. As a result, it was confirmed that compared to stem cells cultured without a sialyllactose, hBMSCs (FIG. 4), hATMSCs (FIG. 5), and hCBMSCs (FIG. 6) cultured after being treated with 3'-sialyllactose, 6'-sialyllactose, and a mixture of 3'-sialyllactose and 6'-sialyllactose had all significantly increased expression at mRNA and protein levels of OCT4, SOX2, and NANOG, which are the marker genes for confirming stemness.

From the test results, it was confirmed that the sialyloligosaccharide treatment before, during, and after culturing of the stem cells may significantly augment stemness of stem cells.

### Example 4. Confirmation of reduction in senescence of stem cells according to 3'-sialyllactose treatment

In Example 4, influence of a sialyloligosaccharide on senescence of stem cells was to be confirmed. Particularly, after treating the hBMSC stem cells with 3'-sialyllactose, changes in expression at mRNA and protein levels of p16 and p53, which are cell senescence factors after subculturing were each measured by qRT-PCT and Western Blot. In particular, total RNAs were isolated from the cells collected after culturing the cells in the same manner as in Example 1, and change in mRNA expression of the isolated total RNAs was confirmed by qRT-PCR using p16 primers (P260189, available from Bioneer) and p53 primers (P250999, available from Bioneer) verified by Bioneer (Daejeon, Korea).

FIG. 7 shows the results of confirming influence of 3'-sialyllactose on senescence of stem cells. As a result, as senescence of hBMSCs subcultured without 3'-sialyllactose treatment proceeded, the expressions at mRNA and protein levels of p16 and p53 increased, but the stem cells subcultured after being treated with 3'-sialyllactose showed very low expression at mRNA and protein levels of p16 and p53, which confirmed decreased expressions of p16 and p53.

Also, in order to confirm a degree of senescence of the stem cells after being treated with 3'-sialyllactose (GeneChem), senescence-associated beta-galactosidase staining was performed on hBMSCs of 10 passages, treated with 100 µM of 3'-sialyllactose. In particular, after fixing the cells in formalin, the resultant washed with PBS, and 1 mg/mL of a β-gal staining solution X-Gal, 40 mM of a citric acid-sodium phosphate buffer solution, 150 mM of NaCl, 2 mM of MgCl₂, 5 mM of potassium ferrocyanide, and 5 mM of potassium ferricyanide (pH 6.0, available from Sigma-Aldrich, St. Louis, USA) were add to the resultant and reacted at 37 °C. Then, degrees of senescence activity of the aged cells (blue, positive) and cells not showing activity of senescence (negative) were confirmed using Eclipse TS100 (available from Nikon USA, Melville, NY, USA).

As a result of the cell senescence analysis using SA-β-gal, the hBMSCs subcultured without 3'-sialyllactose treatment had increased SA-β-gal activity as the senescence proceeded, whereas the stem cells subcultured after 3'-sialyllactose treatment showed low SA-β-gal activity, which confirmed a decrease in the SA-β-gal activity.

From the test results, it was confirmed that the sialyloligosaccharide treatment before, during, and after culturing of the stem cells may significantly reduce senescence of stem cells.

### Example 5. Confirmation of increase in proliferation of stem cells according to 3'-sialyllactose treatment

In Example 5, influence of a sialyloligosaccharide on proliferation of stem cells was to be confirmed. Particularly, hBMSCs having low proliferation ability were treated with 3'-sialyllactose, and changes in expressions of cyclin A2 (CCNA2), which is a gene involved in cell cycle progression and proliferation and cyclin-dependent kinase 2 (CDK2) were confirmed. In particular, total RNAs were isolated from the cells collected after culturing the cells in the same manner as in Example 1, and change in mRNA expression of the isolated total RNAs was confirmed by qRT-PCR using CCNA primers (P212796, available from Bioneer) and CDK2 primers (P2136765, available from Bioneer) verified by Bioneer (Daejeon, Korea).

FIG. 8 shows the results of confirming influence of 3'-sialyllactose on proliferation of hBMSCs. As a result, it was confirmed that expressions of CCNA2 and CDK2 were reduced as the senescence of the stem cells without 3'-sialyllactose treatment proceeded, but expressions of CCNA2 and CDK2 of the stem cells with 3'-sialyllactose treatment significantly increased.

From the test results, it was confirmed that the sialyloligosaccharide treatment before, during, and after culturing of the stem cells may significantly increase proliferation of stem cells.

### Example 6. Confirmation of increase in viability of stem cells according to number of passages according to 3'-sialyllactose treatment

In Example 6, influence of a sialyloligosaccharide on viability of stem cells according to cell subculturing passages was to be confirmed. Particularly, hBMSCs subcultured for 4, 7, 10, and 12 passages were treated with 3'-sialyllactose in a concentration of 100 µM, and the cell viability was confirmed by cell counting kit-8 assay (CCK-8) analysis. In particular, the hBMSCs were seeded in a 96-well plate. Then, the stem cells were treated with 3'-sialyllactose (GeneChem) in a concentration of 100 µM and cultured in a DMEM low glucose medium (HyClone) including 1 % antibiotics free of fetal bovine serum (T&I). Viabilities of the cultured cells were confirmed by the CCK-8 analysis.

FIG. 9 shows the results of confirming influence of 3'-sialyllactose on viability of stem cells. As a result, it was confirmed that the cell viability of the stem cells treated with 3'-sialyllactose significantly increased.

From this test result, it was confirmed that self-renewal of stem cells is deteriorated according to subculturing passages, but sialyloligosaccharide treatment may significantly suppress deterioration of proliferating ability according to subculturing passages.

### Example 7. Confirmation of increase in viability of organoid according to sialyllactose treatment

In Example 7, influence of a sialyloligosaccharide on induced pluripotent stem cells-derived hepatic organoids was to be confirmed. Particularly, after subculturing the hepatic organoids, the organoids were stabilized in a hepatic medium (HM) for a day and treated with 3'-sialyllactose and 6'-sialyllactose in concentrations of 0, 0.1, 1, 10, and 100 µM for 6 days, and then the cell viabilities of the organoids on the 7th day were confirmed by the CCK analysis.

FIG. 10 shows the results of confirming influence of a sialyllactose on cell viability of induced pluripotent stem cells-derived hepatic organoids. As a result, it was confirmed that the cell viability of hepatic organoids treated with 3'-sialyllactose in concentrations of 0.1, 1, 10, and 100 µM and hepatic organoids treated with 6'-sialyllactose in concentrations of 0.1, 1, 10, and 100 µM significantly increased.

From the test results, it was confirmed that the sialyloligosaccharide treatment during culturing of the induced pluripotent stem cells-derived hepatic organoids may significantly increase proliferation of the hepatic organoids.

### Example 8. Confirmation of increase in maturity of organoid according to sialyllactose treatment

In Example 8, influence of a sialyloligosaccharide on maturity of induced pluripotent stem cells-derived hepatic organoids was to be confirmed. Particularly, after subculturing the hepatic organoids, the organoids were stabilized in a hepatic medium (HM) for a day and treated with 3'-sialyllactose and 6'-sialyllactose in concentrations of 0, 0.1, 1, 10, and 100 µM for 6 days, and then total RNAs were isolated from the hepatic organoids on the 7th day. The changes in expressions at mRNA level of CYP3A4, CYP2A7, ALB, and AFP, which are markers of metabolic maturity, were each measured by qRT-PCR, and the expression ratios were compared to confirm the metabolic maturity.

FIGS. 11 and 12 show the results of confirming influence of a sialyllactose on cell maturity of induced pluripotent stem cells-derived hepatic organoids. As a result, it was confirmed that ratios of CYP3A4/CYP3A7 of the hepatic organoids treated with 3'-sialyllactose in concentrations of 1 and 10 µM and ratios of ALB/AFP of the hepatic organoids treated with 3'-sialyllactose in concentrations of 1, 10, and 100 µM were significantly increased and that ratios of ALB/AFP of the hepatic organoids treated with 6'-sialyllactose in concentrations of 0.1 and 100 µM were significantly increased.

From the test results, it was confirmed that the sialyloligosaccharide treatment during culturing of the induced pluripotent stem cells-derived hepatic organoids may significantly increase metabolic maturity of the hepatic organoids.

It will be understood by those skilled in the art that the foregoing description of the present invention is for illustrative purposes only and that those of ordinary skill in the art may readily understand that various changes and modifications may be made without departing from the spirit or essential characteristics of the present invention. Therefore, it should be understood that the embodiments described above are illustrative in all aspects and should not be construed as limiting the scope of the present invention.

## Claims

1. A composition for augmenting stemness of stem cells, the composition comprising a sialyloligosaccharide.

2. The composition of claim 1, wherein the sialyloligosaccharide comprises 3'-sialyllactose, 6'-sialyllactose, 3'-sialyllactosamine, 6'-sialyllactosamine, 3'-sialyl-3-fucosyllactose, sialyllacto-N-tetraose, disialyllactose, or a combination thereof.

3. The composition of claim 1, further comprising a human milk oligosaccharide.

4. The composition of claim 3, wherein the human milk oligosaccharide comprises lacto-N-tetraose, 2'-fucosyllactose, 3-fucosyllactose, lacto-N-neotetraose, LS-tetrasaccharide b, LS-tetrasaccharide c, disialyllacto-N-tetraose, or a combination thereof.

5. The composition of claim 1, wherein the stem cells are induced pluripotent stem cells, embryonic stem cells, or adult stem cells.

6. The composition of claim 5, wherein the adult stem cells are stem cells derived from a tissue selected from the group consisting of umbilical cord, umbilical cord blood, synovial membrane, bone marrow, fat, muscle, nerve, skin, amniotic membrane, and placenta.

7. The composition of claim 1, wherein the stem cells may be cells of autologous, allogeneic or xenogeneic origin.

8. The composition of claim 1, wherein the composition comprises a sialyloligosaccharide in a concentration from about 0.1 µM to about 400 µM.

9. The composition of claim 1, wherein the stemness is augmented by at least one characteristic selected from the group consisting of suppressing cell senescence of stem cells, increasing cell proliferation ability, increasing stem cell functionality, increasing telomerase activity, increasing expression of stem cell factors, increasing protein homeostasis, and increasing cell migration activity.

10. The composition of claim 1, wherein the composition is provided in a medium composition form.

11. The composition of claim 10, wherein the medium is a basal medium selected from the group consisting of Dulbecco's Modified Eagle's Medium (DMEM), 80 % knockout DMEM, Minimal Essential Medium (MEM), Basal Medium Eagle (BME), RPMI 1640, F-10, F-12, DMEM-F12, α-Minimal Essential Medium (α-MEM), Glasgow's Minimal Essential Medium (G-MEM), Iscove's Modified Dulbecco's Medium (IMDM), MacCoy's 5A medium, AmnioMax Medium, and Chang's Medium MesemCult-XF Medium.

12. The composition of claim 10, wherein the medium further comprises an antioxidant selected from the group consisting of selenium, ascorbic acid, vitamin E, catechin, lycopene, beta-carotene, coenzyme Q-10 (CoQ-10), resveratrol, T-BHQ, oltipraz, alnus japonica extract, eicosapentaenoic acid (EPA), docosahexaenoic acid (DHA), and a combination thereof.

13. The composition of claim 10, wherein the medium further comprises an additional component selected from the group consisting of N-acetyl-L-cysteine (NAC), insulin or insulin-like factor, hydrocortisone, dexamethasone, basic fibroblast growth factor (bFGF), heparan sulfate, 2-mercaptoethanol, epidermal growth factor (EFG), B-27, activin A, BMP-4, oncostatin M (OSM), hepatocyte growth factor (HGF), and a combination thereof.

14. The composition of claim 1, wherein the composition is locally administered to a lesion site.

15. The composition of claim 14, wherein the composition is provided in an injectable form.

16. A method of culturing stem cells, the method comprising culturing stem cells in a medium comprising a sialyloligosaccharide.

17. The method of claim 16, wherein the sialyloligosaccharide comprises 3'-sialyllactose, 6'-sialyllactose, 3'-sialyllactosamine, 6'-sialyllactosamine, 3'-sialyl-3-fucosyllactose, sialyllacto-N-tetraose, disialyllactose, or a combination thereof.

18. The method of claim 16, wherein the medium further comprises a human milk oligosaccharide.

19. The method of claim 18, wherein the human milk oligosaccharide comprises lacto-N-tetraose, 2'-fucosyllactose, 3-fucosyllactose, lacto-N-neotetraose, LS-tetrasaccharide b, LS-tetrasaccharide c, disialyllacto-N-tetraose, or a combination thereof.

20. The method of claim 16, wherein the culturing of the stem cells is culturing stem cells in a basal medium selected from the group consisting of Dulbecco's Modified Eagle's Medium (DMEM), 80 % knockout DMEM, Minimal Essential Medium (MEM), Basal Medium Eagle (BME), RPMI 1640, F-10, F-12, DMEM-F12, α-Minimal Essential Medium (a-MEM), Glasgow's Minimal Essential Medium (G-MEM), Iscove's Modified Dulbecco's Medium (IMDM), MacCoy's 5A medium, AmnioMax Medium, and Chang's Medium MesemCult-XF Medium.

21. The method of claim 16, wherein the culturing of the stem cells is culturing stem cells in a medium comprising a sialyloligosaccharide at a concentration in a range of about 0.1 µM to about 400 µM.

22. The method of claim 16, wherein the method maintains or augments the cultured stemness.

23. The method of claim 16, wherein the culturing of the stem cells comprises subculturing stem cells.

24. The method of claim 23, wherein the method maintains the stemness after 3 to 12 passages.

25. A method of augmenting stemness of stem cells, the method comprising adding a sialyloligosaccharide to the stem cells.

26. The method of claim 25, wherein the sialyloligosaccharide comprises 3'-sialyllactose, 6'-sialyllactose, 3'-sialyllactosamine, 6'-sialyllactosamine, 3'-sialyl-3-fucosyllactose, sialyllacto-N-tetraose, disialyllactose, or a combination thereof.

27. The method of claim 25, wherein the adding of the sialyloligosaccharide further comprises adding a human milk oligosaccharide.

28. The method of claim 27, wherein the human milk oligosaccharide comprises lacto-N-tetraose, 2'-fucosyllactose, 3-fucosyllactose, lacto-N-neotetraose, LS-tetrasaccharide b, LS-tetrasaccharide c, disialyllacto-N-tetraose, or a combination thereof.

29. The method of claim 25, wherein the adding of the sialyloligosaccharide is performed *ex vivo* or *in vitro.*

30. The method of claim 25, wherein the adding of the sialyloligosaccharide is performed by adding a sialyloligosaccharide at a concentration in a range of about 0.1 µM to about 400 µM.

31. The method of claim 25, wherein the stemness is augmented by at least one characteristic selected from the group consisting of suppressing cell senescence of stem cells, increasing cell proliferation ability, increasing stem cell functionality, increasing telomerase activity, increasing expression of stem cell factors, increasing protein homeostasis, and increasing cell migration activity.

32. A stem cell having augmented stemness using the method of any one of claims 16 to 24.

33. A composition for cell therapy, the composition comprising the stem cell of claim 32 or a dilution thereof as an active ingredient.

34. A composition for culturing hepatic organoid, the composition comprising a sialyloligosaccharide.

35. The composition of claim 34, wherein the hepatic organoid is derived from induced pluripotent stem cells, embryonic stem cells, or adult stem cells.

36. The composition of claim 34, wherein the sialyloligosaccharide comprises 3'-sialyllactose, 6'-sialyllactose, 3'-sialyllactosamine, 6'-sialyllactosamine, 3'-sialyl-3-fucosyllactose, sialyllacto-N-tetraose, disialyllactose, or a combination thereof.
